# EUROPEAN PATENT APPLICATION

(11) **EP 0 808 897 A1**
(43) Date of publication of application: **26.11.1997**
(21) Application number: 96401099.5
(22) Date of filing: 21.05.1996
(51) Int. Cl.: C12N 5/08, A61K 35/14, C07K 16/46

(54) **New antigen presenting cells, a process for preparing the same and their use as cellular vaccines**

(71) Applicant: I.D.M. IMMUNO-DESIGNED MOLECULES, 75011 Paris (FR)
(72) Inventor: Chokri, Mohamed, 67000 Strasbourg (FR); Bartholeyns, Jacques, 91440 Bures-sur-Yvette (FR); Romet-Lemonne, 75003 Paris (FR)
(74) Representative: Grosset-Fournier, Chantal Catherine

(57) **Abstract**

The invention relates to macrophages characterized in that they have the following properties:
- they present on their surface :
   * antigen CD14 with a mean intensity of about 20 to about 200,
   * antigen CD64 with a mean intensity of about 20 to about 200,
- they are substantially devoid of the surface antigens CD1a and CD1c,
   the presence and mean intensities respectively of CD14, CD64 and the absence of CD1a and CD1c being for instance determined by immunofluorescence staining and flow cytometry analysis,
- they present a phagocytosis property such as determined by the following test:
   said phagocytosis capacity being evaluated by an uptake of formalin fixed yeast, for example by culturing macrophages for 2 hours, adding yeast in 1/10 macrophages/yeast ratio and incubating at 37°C, 5% CO₂ atmosphere for 2-3 hours fixing by the May-Grünwald-Giems a (MGG) staining, and the percentage of phagocytic macrophages being quantified for instance by microscopic analysis,
- they have the property of stimulating the proliferation of allogenic lymphocytes such as determined by the following test :
   allogenic primary mixed lymphocytes reaction (MLR) was carried out in 96-well microtiter plates by adding different numbers (2x10³ to 2x10⁵ in 100 µl medium/well) of macrophages to 2x10⁵ in 100 µl medium/well of allogenic T cells purified from buffy coats and after 5 days incubation at 37°C, cell proliferation was assessed by a colorimetric method, such as the hydrolysis of tetrazolium salt WST-1 (Boehringer Mannheim, Germany), (slightly red) to Formozan (dark red).

## Description

The invention relates to new antigen presenting cells, a process for preparing the same and their use as cellular vaccines.

Macrophages are recognized since their description by Metchnikoff (Immunity in Infective Diseases, Cambridge Press, 1905) as cells which very effectively phagocytose (interiorise) and digest exogenous particles (antigens, cell debris, bacteria). They also secrete a variety of immunoeffector monokines. They have therefore a central role in initiating the non-specific and the specific immune responses. The recovery of large quantities of human macrophages differentiated in culture from blood monocytes has already been described (International application N° PCT/EP93/01232, refs. 1-6). These macrophages express typical antigens and functions and have been fully characterized (refs. 7-14).

Macrophages activated in the presence of IFNγ (Macrophage Activated Killers : MAK) elicited selective cytostasis and cytotoxicity for a large number of human tumors even at low effector/target ratio. In murine models, murine activated macrophages given locally in the tumor or its vicinity infiltrated the tumor mass, inhibited tumor growth and decreased metastatic development. Human macrophages also inhibited the growth of human tumors engrafted in nude or SCID mice ; this effect was achieved after local or systemic injection of a low number of macrophages (less than 1 million MAK) for mice with macroscopic tumors (ref. 15-20).

Patients with metastatic cancer were infused systemically or intraperitoneally with 10⁸ to 4x10⁹ autologous MAK. Tumoricidal monocytes (AKM) were also infused intraperitoneally in patients with colorectal carcinomas. The clinical tolerance of MAK was excellent with minor side effects such as low grade fever and chills and no autoimmune nor acute phase reactivity. No complete antitumoral response was reported ; improved prognosis and prolonged disease free intervals were described after intraperitoneal injection of AKM, while tumor necrosis, stabilisation, reduction of ascitic fluid and change in chemioresistance were seen after MAK therapy (refs. 21 - 26).

The recognized limitation of these macrophages is that they are not very potent in the priming of a specific immune response against a specific exogenous antigen or tumor by stimulation of MHC class I restricted cytotoxic T lymphocytes (CD8 +). They are more efficient in presenting antigens in the context of MHC class II molecules to T helper lymphocytes (CD4 +).

However these macrophages have the potential to process and present soluble antigens by the exogenous pathway of phagocytes but high concentrations of proteins or antigens are required (refs. 38 - 42, 45 - 47, 50).

Cells expressing these functions of phagocytosis, digestion, processing, and presentation at a high level would be required for the development of cellular vaccines.

Dendritic cells are characterized by their morphology (dendrites) and a few membrane antigens, and are considered as professional antigen - presenting cells resident in tissues. They are the most potent cells for the stimulation of primary T - lymphocyte immune responses (refs. 43 - 44, 47 - 49, + Dendritic cells in fundamental and clinical immunology. 1995, Plenum Press N.Y., Banchereau and Schmitt Editors).

Dendritic cell precursors arise from bone marrow and can be found in blood and lymph. Dendritic cells derived from these origins can be obtained by culture in the presence of GM - CSF + IL4 + IL13 or + TNF. They will then exhibit differences related to their maturation state and microenvironment.

The dendritic cells which can be derived from blood are most potent to induce allogenic mixed lymphocyte reactions and to stimulate naive T lymphocytes. However, these classical dendritic cells are technically relatively difficult to obtain and are poorly phagocytozing.

In contrast to macrophages, they do not express or have only a dim expression of CD14 and CD64 (high affinity Fγ receptor).

To circumvent the problem of poor phagocytoses and processing of particular antigens by dendritic cells, these cells have been pulsed with small peptides fixed on MHC-I molecules to induce primary immune reaction and vaccination against new antigenic peptides (ref. 51).

Obtaining dendritic cells by *in vitro* differentiation requires the presence of GM-CSF and of a second cytokine that can be TNF, IL 4 (ref. 44) or IL 13 (ref. 49).

One of the aims of the invention is to provide cells with high phagocytosis, and efficient antigen presentation.

Another aim of the invention is to provide very potent antigen presenting cells derived from human blood monocytes.

Another aim of the invention is to provide cells presenting the CD64 high affinity Fc receptor, allowing targeting and increased antigen presentation with the use of bispecific antibodies (refs. 27 - 31, 37).

Another aim of the invention is to provide cells which can be transfected with cDNA to be used in gene therapy (ref. 32 - 36).

Another aim of the invention is to provide a method allowing the recovery from human blood of cells of the macrophage lineage with high phagocytosis, digestive activity, processing MHC class I and class II antigen presentation (ref. 46, 50, 52).

Another aim of the invention is to provide a reproducible method allowing to obtain the above defined cells, said process not requiring combination of exogenous cytokines.

Another aim of the invention is to provide cellular vaccines demonstrating the high phagocytosis, processing, MHC-II peptide presentation of the macrophages and also the potent MHC-I T lymphocyte stimulation with high level of accessory molecules for presentation of the dendritic cells.

The invention relates to macrophages which have the following properties:
- they present on their surface :
   * antigen CD14 with a mean intensity of about 20 to about 200,
   * antigen CD64 with a mean intensity of about 20 to about 200,
- they are substantially devoid of the surface antigens CD1a and CD1c,
   the presence and mean intensities respectively of CD14, CD64 and the absence of CD1a and CD1c being for instance determined by immunofluorescence staining and flow cytometry analysis,
- they present a phagocytosis property such as determined by the following test:
   said phagocytosis capacity being evaluated by an uptake of formalin fixed yeast, for example by culturing macrophages for 2 hours, adding yeast in 1/10 macrophage/yeast ratio and incubating at 37°C, 5% CO₂ atmosphere for 2-3 hours fixing by the May-Grünwald-Giemsa (MGG) staining, and the percentage of phagocytic macrophages being quantified for instance by microscopic analysis,
- they have the property of stimulating the proliferation of allogenic lymphocytes such as determined by the following test :
   allogenic primary mixed lymphocytes reaction (MLR) was carried out in 96-well microtiter plates by adding increasing numbers (2x10³ to 2x10⁵) in 100 µl medium/well of macrophages to 2x10⁵ in 100 µl medium/well of allogenic T cells purified from buffy coats and after 5 days incubation at 37°C, cell proliferation was assessed by a colorimetric method, such as the hydrolysis of tetrazolium salt WST-1 (Boehringer Mannheim, Germany), (slightly red) to Formozan (dark red).

In the context of the present invention, the expression "macrophages" designates not only macrophages but antigen presenting cells which derive from monocytes and which will be hereafter designated by MD-APC.

The expression "substantially devoid of surface antigens CD1a and CD1c" means either that there is no such surface antigens or that there is only a dim intensity for these surface antigens, with said dim intensity corresponding to about 10 times less than the intensity obtained in the presence of such surface antigens, as determined in immunofluorescence analysis, or with said intensity being lower than 20.

In the following of the text, it will be often referred to "the absence of surface antigen CD1a and CD1c", which is to be understood as "substantially devoid of such antigens" as explained above.

The presence of antigen CD14 and CD64 corresponds to an intensity of at least 20 to 200 for the CD14 and 20 to 200 for the CD64.

The absence of CD1a and CD1c corresponds to an intensity lower than 20.

The invention also relates to macrophages which present, on their surface, antigen MHC-II with a mean intensity of about 100 to about 400, such as determined by immunofluorescence staining and flow cytometry analysis.

The invention also relates to macrophages which are substantially devoid of surface antigen CD83, such as determined by immunofluorescence staining and flow cytometry analysis.

The expression "substantially devoid of surface antigen CD83" corresponds to an intensity lower than 20.

The macrophages of the invention present adherent properties such as determined by the following test:
the macrophages are cultured for 2 h in culture medium (I.M.D.M. or R.P.M.I.) on plastic flasks and the percentage (%) of adherent cells is quantified for instance by microscopic analysis.

The culture media I.M.D.M. and R.P.M.I. are commercially available.

The invention relates to a macrophage culture wherein :
- about 20% to about 100% of the macrophages present antigen CD14 on their surface,
- about 20% to about 100% of the macrophages present antigen CD64 on their surface,
- about 80% to about 100% of the macrophages present antigen MHC-II on their surface,
- about 70% to about 100% of the macrophages present adherent properties,
- about 30% to about 100% of the macrophages present a phagocytosis property,
each macrophage having the above-mentioned properties being such that said properties are expressed according to the intensities as specified above.

The invention also relates to a process for preparing a composition of macrophages which comprises the culture of mononuclear cells in a culture medium containing histamine or histamine agonist (H₁ in action) and a H₂ antagonist, in combination or not with "additional" GM-CSF.

An example of histamine agonist is 2 methyl-histamine.

The expression "additional" corresponds to the fact that there is no GM-CSF added to the culture in standard condition, but this does not exclude the fact that exogenous GM-CSF could be added in the culture to increase yield and functions of MD-APCs obtained.

As example of H₂ antagonist, one may cite cimetidine but also tiotidine, burimamide, metiamide, ranitidine.

The invention also relates to a process wherein the culture medium contains histamine and cimetidine or a H₂ antagonist without "additional" GM-CSF, histamine being present at a concentration of about 10⁻² M to about 10⁻⁶ M, preferably of about 10⁻⁴ M, and cimetidine or the H₂ antagonist being present at a concentration of about 10⁻⁴ M to about 10⁻⁹ M, preferably of about 10⁻⁶ M.

When the concentration of histamine or cimetidine is lower than the lower value of the given range, there is substantially no effect, i.e. less than 10% difference with cells cultured in the absence of histamine and cimetidine.

When the concentration of histamine or cimetidine is higher than the higher value of the given range, the culture medium becomes toxic.

When no additional GM-CSF is incorporated into the culture medium, GM-CSF secreted *in situ* by the macrophages can be present at a concentration of about 5 to about 500 U/ml.

The invention also relates to a process wherein the culture medium contains histamine and cimetidine or a H₂ antagonist, in combination with "additional" GM-CSF, histamine being present at a concentration of about 10⁻² M to about 10⁻⁶ M, preferably of about 10⁻⁴ M, cimetidine or the H₂ antagonist being present at a concentration of about 10⁻⁴ M to about 10⁻⁹ M, preferably of about 10⁻⁶ M, and additional GM-CSF being present at a concentration of about 50 U/ml to about 1000 U/ml, preferably of about 500 U/ml.

When additional GM-CSF is incorporated into the culture, then the total concentration of GM-CSF is from about 50 U/ml to about 2000 U/ml, and preferably from about 50 U/ml to about 500 U/ml.

According to a particular embodiment of the invention, the culture medium does not comprise the following elements : exogenous cytokines such as IL4, IL10, IL13, TNF.

The invention also relates to a process comprising :
- isolation of leukocytes, from healthy donors or from patients, from peripheral blood by apheresis and removal of platelets and anticoagulant from the apheresis product,
- isolation of mononuclear cells (monocytes + lymphocytes) from red cells and granulocytes in order to have less than 10% granulocytes and less than 5% red cells,
- culture of the mononuclear cells obtained at the previous stage by placing them in an appropriate culture medium containing histamine or an agonist of histamine, an H₂ antagonist, such as cimetidine, in combination or not with GM-CSF, for a time sufficient to obtain differentiated macrophages, preferably for about 5 to 15 days, and possibly separating the macrophages from the lymphocytes, and recovering the macrophages or the macrophages and lymphocytes.

The invention also relates to a process wherein the culture medium of macrophages is added
- with crude antigens, for instance autologous tumor membrane, killed tumoral cells, bacterial capsides, viral homogenates cleared from nucleic acids,
- specific peptides against which an immune response is desired,
- cDNA or genetic material linked to vectors (for example gluconated polylysine) to allow transfection of the macrophage with material coding for the relevant peptide or protein to be presented on the macrophage membrane and against which an immune response is desired,
- or bispecific antibodies targeting on the one side, FcγRI (CD64) surface antigen of the macrophage and, on the other side, a relevant antigen against which an immune response is desired.

The invention also relates to macrophages liable to be obtained according to the process of the invention described hereabove.

The invention also relates to pharmaceutical compositions containing as active substance, macrophages according to the invention.

The invention also relates to cellular vaccine compositions containing as active substance, macrophages according to the invention.

The invention also relates to the medium containing elements necessary for the growth and differentiation of monocytes into macrophages according to the invention, and in addition containing histamine, cimetidine in combination or not with GM-CSF.

The invention also relates to a a cell processor or a kit containing :
- means for the recovery of lymphocytes and monocytes free of contaminants,
- appropriate buffer and wash solutions and possibly appropriate means for the conservation of macrophages,
- means for preparing a culture for the monocytes and possibly the lymphocytes and containing histamine, cimetidine or a H₂ antagonist in combination or not with GM-CSF,
- possibly means for transfection of cultured cells and means for targeting antigens to macrophages.

Regarding the conservation of macrophages, it can include freezing means for example in 10% glycerol or D.M.S.O. (dimethyl sulfoxyde) in the presence of autologous or AB⁺ serum.

The invention also relates to a a cell processor or a kit as described above which contains :
- means for recovering and centrifuging blood to obtain a leukocyte concentrate,
- means for separating lymphocytes and monocytes from the other white cells and for eliminating the contaminating red cells,
- culture medium for macrophages and possibly lymphocytes with complements and particularly histamine and cimetidine or a H₂ antagonist, in combination or not with GM-CSF,
- appropriate means for the conservation of macrophages,
- appropriate buffer and wash solution.

The invention also relates to products containing macrophages according to the invention, and lymphocytes, as a combined preparation for simultaneous, separate or sequential use in cell therapy.

The invention also relates to products as described hereabove which contain the macrophages and the lymphocytes in a ratio of at least 20% to 50% of macrophages expressed in cell number.

The invention also relates to bispecific antibodies liable to recognize an antigen of a macrophage of the invention and an antigen of a tumoral cell or of a pathogen which is to be targetted to said macrophage.

The invention also relates to a method for the clinical treatment, comprising the administration of an appropriate amount of macrophages according to the invention, and preferably in an amount of about 10⁸ to about 5x10⁹ macrophages.

The invention also relates to a method for the treatment of any disorder, comprising the administration of lymphocytes from the culture in an amount of about 4x10⁹ to about 10x10⁹ lymphocytes.

The invention also relates to the use of an agonist of histamine receptor, in particular histamine, and a H₂ antagonist, in particular cimetidine, in combination or not with GM-CSF, for the preparation of macrophages having the following properties :
- they present on their surface :
   * antigen CD14 with a mean intensity of about 20 to about 200,
   * antigen CD64 with a mean intensity of about 20 to about 200,
- they are substantially devoid of the surface antigens CD1a and CD1c,
   the presence and mean intensities respectively of CD14, CD64 and the absence of CD1a and CD1c being for instance determined by immunofluorescence staining and flow cytometry analysis,
- they present a phagocytosis property such as determined by the following test:
   said phagocytosis capacity being evaluated by an uptake of formalin fixed yeast, for example by culturing macrophages for 2 hours to select adherent cells, adding yeast in 1/10 macrophages to yeast ratio and incubating at 37°C, 5% CO₂ atmosphere for 2-3 hours fixing by the May-Grünwald-Giemsa (MGG) staining, and the percentage of phagocytic macrophages being quantified for instance by microscopic analysis,
- they have the property of stimulating the proliferation of allogenic lymphocytes such as determined by the following test:
   allogenic primary mixed lymphocytes reaction (MLR) was carried out in 96-well microtiter plates by adding different numbers 2x10³ to 2x10⁵ in 100 µl medium/well of macrophages to 2x10⁵ in 100 µl medium/well of allogenic T cells purified from buffy coats and after 5 days incubation at 37°C, cell proliferation was assessed by a colorimetric method, such as the cleavage of tetrazolium salt WST-1 (slightly red) to Formozan (dark red).

The monocytes derived antigens presenting cells of the invention and the macrophages can be obtained as follows :
a) Isolation of leukocytes from blood by apheresis and elimination of platelets, by centrifugation. If cells collected have < 10% granulocytes and haematocrit < 5%, they can be seeded as such in culture. Otherwise mononuclear cells have to be prepared first by centrifugation on Ficoll Paque of density 1,077.
b) Mononuclear cells are then cultured for 5 to 15 days in hydrophobic bags ethylene vinyl acetate (E.V.A., Stedim) or polypropylene (Life Cell, Baxter), at 37°C, 5% CO₂. Cells are seeded at 5.10⁶/ml in I.M.D.M. (as previous) or equivalent medium supplemented with indomethacin (5x10⁻⁶M) mercaptoethanol (3x10⁻⁵M), non essential amino-acids (1%, Gibco) and 2 to 5% of reconstituted autologous or *AB*⁺ serum,
   . with GM-CSF, 500 U/ml, histamine (10⁻⁴ M), cimetidine (10⁻⁶ M) or another H₂ antagonist of histamine, or with histamine, cimetidine in the absence of any cytokines
   . and exogenous antigens, peptides or transfectants (cDNA + vector) coding for the relevant antigen.
c) After culture, the specific monocyte derived antigen presenting cells (MD-APCs) are centrifuged, washed and resuspended for injection in the patient, to induce humoral and cellular immune response against the antigen.

The specificity of the cellular vaccine is achieved during the *in vitro* culture according to one of the following items.
a) culture of MD-APCs as explained above in b), in the presence of crude antigens, for example autologous tumor membrane, bacterial capsides, viral homogenates cleared from nucleic acids ;
b) culture of MD-APCs as explained above in b), in the presence of specific peptides against which immune response would be beneficial,
c) or culture of MD-APCs as explained above in b), in the presence of cDNA or genetic material linked to vectors (for example gluconated polyphysine) to allow transfection of MD-APCs with material coding for the relevant peptide or protein to be presented on the membrane.

In order to obtain specific cellular vaccine, it is also possible at the end of the differentiation stage of the macrophage culture to add bispecific antibodies targeting FcγRI (CD64) of MD-APCs on one side and the relevant antigen on the other side.

According to a preferred embodiment, the process of the invention comprises the following steps:
- isolation of leukocytes from blood of healthy subjects or patients by apheresis, to obtain the apheresis products (i.e. concentrated leukocytes),
- platelet elimination, for instance by centrifugation of the apheresis products, to obtain a leukocyte enriched product,
- separation, in the leukocyte enriched products, of the mononuclear cells on one hand, and of the contaminating red blood cells and granulocytes on the other hand,
- culture of the mononuclear cells (monocytes + lymphocytes) in a medium containing histamine and cimetidine and GM-CSF for about 5 to 15 days, to obtain differentiated monocyte derived antigen presenting cells (MD-APCs).

The lymphocytes can be separated from the monocytes before the culture step.

The lymphocytes can be separated from the MD-APCs after the culture.

In the process of the invention, histamine is used at a concentration of 10⁻² M to about 10⁻⁶ M, preferably of about 10⁻⁴ M.

In the process of the invention, GM-CSF is used at a concentration of about 50 to about 1000 U/ml, particularly of about 100 to about 500 U/ml.

In the process of the invention, the culture medium is RPMI, IMDM, MEM, or DMEM.

These media are commercially available.

Advantageously, the culture medium contains indomethacin (or another cyclo-oxygenase inhibitor) or/and cimetidine (an histamine H₂ antagonist).

An advantageous process for preparing the macrophages of the invention is the following:

### Apheresis

Leukocytes from healthy subjects or from patients are isolated from peripheral blood by apheresis using the Cobe Spectra continous-flow blood cell separators keeping granulocytes contamination very low (< 10% and less than 5% red cells). The apheresis product is centrifuged for 10 min at 280 g in order to reduce platelet contamination. The platelet-enriched plasma is removed and leukocyte pellet resuspended in a phosphate buffer solution (PBS) containing 0.1% glucose, 0.17% PO₃HNa₂, 2H₂O, 0.27% PO₃H₂Na, 0.14% NH₄Cl, 0.78% NaCl (solution TS745 laboratoire Bruneau, France).

The enriched leukocyte pellet is obtained with an average of 7 to 1.5x10¹⁰ leukocytes (50% of mononuclear cells).

### Isolation of mononuclear cells

If the collected leukocytes have more than 10% granulocytes contamination and/or 5% hematrocrite, human mononuclear cells are separated from red blood cells and from contaminating granulocytes, by 15 min centrifugation at 1000 g on a COBE 2991 or Stericell cell processor using Ficoll Paque of density 1.077 (Pharmacia). After 3 washings in phosphate buffered saline solution without calcium and magnesium, the monocytes are obtained with about 20% to 50% purity as shown by channelyser analysis (Coulter Margency-France).

### Culture

Differentiated human MD-APC are obtained by 5-15 days in culture of mononuclear cells in hydrophobic bags in E.V.A. (Ethylene Vinyl Acetate, STEDIM, Aubagne) or polypropylene (life cell-Baxter) at 37°C and 5% CO₂, 95% humidified atmosphere. Total mononuclear cells are seeded at 5x10⁶ cells/ml in Iscove modified medium (***I.M.D.M.***, Gibco) or equivalent medium supplemented by penicillin (100 UI/ml), streptomycine (100 µg/ml), L-glutamine (2 mM, Gibco), pyruvic acid (2 mM, Gibco), Indomethacin (5x10⁻⁶ M, Sigma), cimetidine (10⁻⁸ to 10⁻⁴ M), histamine (10⁻⁶ to 10⁻² M), mercaptoethanol (3x10⁻⁵ M, Gibco) non-essential amino-acids (1%, Gibco) and 2-5% of autologous or AB serum. The addition of GM-CSF (500 U/ml, SANDOZ) was done in comparative experiment.

According to a preferred embodiment, the process of the invention is such that killed tumoral cells are added into the culture medium simultaneously with monocytes, both cells coming preferably from the same patient, preferably at the ratio of about 1 million of killed tumoral cells/ml, with said killed tumoral cells being processed at the same time as macrophages.

The killed tumoral cells can then be processed simultaneously with the leukocytes, in an amount of about 1x10⁶/ml.

This process allows to obtain MD-APCs and lymphocytes specific for the tumor, inducing very efficiently *in vivo* an immune response to these specific tumor cells.

The invention also relates to MD-APCs liable to be obtained according to the above-defined process.

The invention also relates to pharmaceutical compositions containing, as active substance, MD-APCs as defined above.

The invention also relates to a medium containing elements necessary for the growth and differentiation of monocytes into MD-APCs of the invention, and in addition histamine and GM-CSF.

The monocyte derived antigens presenting cells of the invention can be part of a cell processor or a kit containing:
- means for the recovery of lymphocytes and monocytes free of contaminants;
- appropriate buffer and wash solutions, and possibly appropriate means for the conservation of MD-APCs ;
- means for preparing a culture medium for the monocytes and possibly the lymphocytes and containing histamine and/or GM-CSF;

According to an advantageous embodiment of the invention, the cell processor or kit contains:
- means for recovering and centrifuging blood to obtain a leukocyte concentrate;
- means for separating lymphocytes and monocytes from the other white cells and for eliminating the contaminating red cells;
- culture medium for macrophages and possibly lymphocytes with complements and particularly and/or GM-CSF and possibly indomethacin and/or cimetidine;
- appropriate means for the conservation of the MD-APCs ;
- appropriate buffer and wash solutions ;

The invention also relates to products containing MD-APCs according to the invention, and lymphocytes, as a combined preparation for simultaneous, separate or sequential use in adoptive immunotherapy.

According to an advantageous embodiment, the products of the invention as defined above are characterised in that they contain the MD-APCs and the lymphocytes in a ratio of at least 20% to 50% of MD-APCs expressed in cell number.

In this embodiment, the MD-APCs and the lymphocytes are both injected to a patient.

The invention also relates to bispecific antibodies liable to recognize an antigen of a MD-APC of the invention, mainly FCγRI (CD 64) and an antigen of a relevant antigen.

The bispecific antibodies can be prepared as described in Chokri et al. Res. Immunol. 143 (1992).

The bispecific antibodies can be injected at the same time as the MD-APCs of the invention, or can be pre-incubated with MD-APCs before injection.

The invention also relates to a method for the treatment of cancer and pathogens comprising the administration of an appropriate amount of MD-APCs according to the invention, and preferably in an amount of about 1x10⁸ to about 5x10⁹ MD-APCs.

### Description of the Figure :

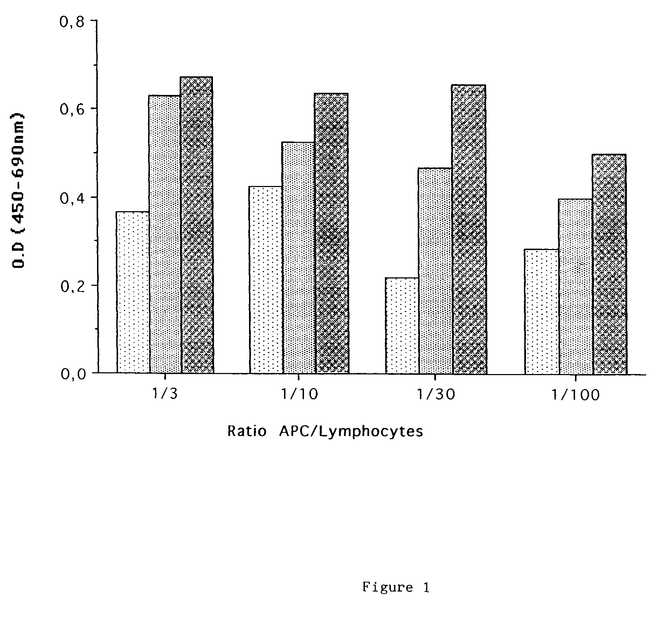

Figure 1 represents the allogenic T cell proliferation induced by MD-APCs of the invention recovered in the presence of histamine (10⁻⁴ M) and cimetidine (10⁻⁶ M) as adjuvant, with or without GM-CSF (500 U/ml) comparing to standard macrophages produced only in the presence of the GM-CSF (500 U/ml).

In Figure 1, the optical density (450-690 nm) has been plotted against the ratio between the MD-APCs of the invention and the responder lymphocyte cells.

The clear dotted bars correspond to the presence of GM-CSF at 500 U/ml; the lighter grey bars correspond to the presence of histamine (10⁻⁴ M) and cimetidine (10⁻⁶ M).

The darker grey bars correspond to the presence of GM-CSF (500 U/ml), in combination with histamine (10⁻⁴ M) and cimetidine (10⁻⁶ M).

The results show that the capacity of the MD-APCs to stimulate the proliferation of allogenic lymphocytes is strongly increased. The stimulation of allogenic proliferation of lymphocytes by MD-APCs is very potent (at least 200% increase with respect to standard macrophages). The combination of histamine/cimetidine effect with GM-CSF can potentiate this activity (maximum of 300% increase with respect to the induction by macrophages).

### EXAMPLE : Preparation of MD-APCs according to the invention:

### 1- Apheresis:

Leukocytes from healthy donors or from patients are isolated from peripheral blood by apheresis using COBE spectra blood cell separator keeping granulocytes contamination the lowest possible. The apheresis product is diluted in a phosphate buffered solution (PBS) (final volume = 450 ml). The apheresis product is centrifuged for 10 min at 280 g to remove platelets and anticoagulant.

### 2- Isolation of mononuclear cells:

If the collected cells have > 10% granulocytes contamination and/or > 5% haematocrit, they should be centrifuged over Ficoll (density = 1.077) to remove red cells and granulocytes.

### 3- Culture

The monocyte derived antigen presenting cells (MD-APCs) are obtained after 5 to 15 days in culture of mononuclear cells in hydrophobic bags in EVA (Ethylene Vinyl Acetate /STEDIM) or equivalent bags (Teflon) at 37°C and 5% CO₂ in humidified atmosphere. The mononuclear cells are seeded at 5x10⁶ cells/ml in Iscove modified medium (I.M.D.M. - Gibco) or equivalent supplemented by (see PCT/EP93 page 7) the addition of histamine (10⁻⁴ M) or analogues and cimetidine (10⁻⁶ M) or similar H₂ antagonist, in the presence or not of GM-CSF (500 U/ml).

### 4- Characterization and functionality

### a- Flow cytometry

After the maturation of MD-APCs, the phenotype analysis of the obtained cells was performed by flow cytometry using murine FITC or P.E labelled monoclonal antibodies directed against membrane proteins.

### b- Mixed lymphocytes reactions (MLR)

To evaluate the induction of T cell response to MD-APCs, allogenic primary MLR was carried out in 96-Well microtiter plates by adding different numbers of MD-APCs to 2x10⁵ allogenic T cells purified from buffy coats. After 5 days at 37°C, cell proliferation was assessed by a colorimetric methods such as WST-1.

### c- Phagocytosis

The phagocytic capacity of the different cells obtained was evaluated by uptake of formalin fixed yeast. Briefly the cells were cultured for 2 hours to select adherent cells. The yeast was added at 1/10 macrophage/yeast ratio and incubated in 37°C, 5% CO₂ atmosphere for 2-3 h and then fixed by the May-Grünwald-Giemsa (MGG) staining. The percentage of phagocytic cells was quantified by microscopic analysis.

The results are gathered in table 1, table 2 and table 3.

**Table 1**

| Yield (% of cells) differentiated in culture | | |
|---|---|---|
| Time of culture | (a) Hist/Cim | (b) Hist.Cim/GM |
| Day 4 | 71% | 78% |
| Day 7 | 49% | 48% |
| Day 11 | 19% | 31% |

Table 1 shows that there is a recovery of a large quantity of MD-APCs after 5 to 11 days of culture of mononuclear cells in hydrophobic bags, in the presence of histamine (10⁻⁴ M) and cimetidine (10⁻⁶ M) (a) and additional GM-CSF (500 U/ml) (b) as adjuvant. In comparison with production of mature macrophages in standard conditions, the recovery of MD-APC cells is in the same order.

**Table 2**

| Phenotype of MD-APCs produced under various conditions after 6 days of culture | | | | |
|---|---|---|---|---|
| Surface Ag | (a) Hist/Cim | | (b) Hist.Cim/GM-CSF | |
| | % | MIF | % | MIF |
| CD1a | N | | N | |
| CD1c | N | | N | |
| CD83 | N | | N | |
| CD14 | 95 | 114 | 95 | 92 |
| CD 54 | 96 | 40 | 96 | 40 |
| CD 58 | 97 | 41 | 97 | 31 |
| CD 64 | 43 | 35 | 91 | 46 |
| HLA-DR | 92 | 350 | 96 | 400 |
| MIF = Mean Intensity Fluorescence. % = percentage of positive cells. N = Negative or weak signal. | | | | |

This table corresponds to the immunofluorescence profile analysis by flow cytometry of MD-APCs generated in culture (6 days) under different conditions. The cells obtained under histamine (10⁻⁴ M) and cimetidine (10⁻⁶ M) conditions (a) are positive for macrophage markers (CD14, CD64 and HLA-DR). The combination with GM-CSF (b) does not change the phenotypic profile of the MD-APCs. They also clearly express CD54 and CD58. The CD80 (B7.1) and CD86 (B7.2) are also expressed on their membrane but in lower level. In contrast, CD1a, CD1c and CD83 which are positive markers of dendritic cells, are weakly expressed by the MD-APCs.

From these data, it can be confirmed that the antigen presenting cells generated in the culture system of the invention are different from dendritic cells.

**Table 3**

| % of phagocytic cells after 3h contact with fixed yeast | | |
|---|---|---|
| Number of intracellular Yeast | (a) Hist/cim | (b) Hist.Cim/GM-CSF |
| 0 | 42% | 20% |
| 1-5 | 43.6% | 43% |
| 6-10 | 11% | 27.5% |
| > 10 | 3.4% | 9.5% |

The MD-APCs are tested for the phagocytic activity using formalin fixed yeast. After 3 h incubation and MGG (May Grümwald Giemsa) staining, the intracellular particles are quantified by microscopic analysis. The MD-APCs generated in the presence of histamine/cimetidine (a) present an important capacity of phagocytosis (60%) and this capacity is increased in the presence of GM-CSF in the culture (80%) (b).

From the above-mentioned results, it is shown that human mononuclear cells cultured in the presence of histamine and cimetidine, in combination or not with GM-CSF mainly for one week, differentiate into mature antigen presenting cells. During the culture, these cells acquire a high level of accessory functions like.

The results of the invention indicate that the MD-APCs obtained in the culture system of the invention are different from the dendritic cells (DC), since DC have been described as FC (CD64) receptor negative, poorly adherent and non-phagocytic cells, possessing only a small number of lysosomes.

In contrast the MD-APCs of the invention:
(a) show a high adherence capacity,
(b) show an important phagocytic and processing activity,
(c) express high level CD14, CD64 and HLA-DR membrane antigens and a very low level of CD1a and CD1c, which is strongly expressed by dendritic cells,
(d) are also positive for CD54, CD58, CD80 and CD86 membrane antigens,
(e) stimulate T-cell proliferation in allogenic MLR reaction, and this is in a far better way than macrophages obtained according to techniques of the prior art.

Table 4 hereafter gathers the comparative characterization of MD-APC of the invention on the one hand, and of dendritic cells on the other hand.

**Table 4**

| Comparative characterization of Antigen Presenting Cells: | | | | |
|---|---|---|---|---|
| Dendritic Cells | | | MD-APC | |
| | | | % cells | Intensity |
| **Phenotype** | | | | |
| CD1a | + | - | 0 | |
| CD1c | + | - | 0 | |
| CD 83 | + | - | 0 | |
| CD14 | +/- | + | 40 to 100 | 80 to 200 |
| CD64 | - | + | 25 to 100 | 20 to 200 |
| CMH-II | +++ | +++ | 80 to 100 | 100 to 400 |

| **Functions:** | | | | |
|---|---|---|---|---|
| Adherence | - | + + | 70 to 90 % | |
| Phagocytosis | - | + + | 30 to 80 % | |
| Stimulation of MLR | +++ | ++ | | |

## Claims

1. Macrophages which have the following properties :
- they present on their surface :
* antigen CD14 with a mean intensity of about 20 to about 200,
* antigen CD64 with a mean intensity of about 20 to about 200,
- they are substantially devoid of the surface antigens CD1a and CD1c,
the presence and mean intensities respectively of CD14, CD64 and the absence of CD1a and CD1c being for instance determined by immunofluorescence staining and flow cytometry analysis,
- they present a phagocytosis property such as determined by the following test:
said phagocytosis capacity being evaluated by an uptake of formalin fixed yeast, for example by culturing macrophages for 2 hours, adding yeast in 1/10 macrophages/yeast ratio and incubating at 37°C, 5% CO₂ atmosphere for 2-3 hours fixing by the May-Grünwald-Giemsa (MGG) staining, and the percentage of phagocytic macrophages being quantified for instance by microscopic analysis,
- they have the property of stimulating the proliferation of allogenic lymphocytes such as determined by the following test :
allogenic primary mixed lymphocytes reaction (MLR) was carried out in 96-well microtiter plates by adding different numbers (2x10³ to 2x10⁵ in 100 µl medium/well) of macrophages to 2x10⁵ in 100 µl medium/well of allogenic T cells purified from buffy coats and after 5 days incubation at 37°C, cell proliferation was assessed by a colorimetric method, such as the hydrolysis of tetrazolium salt WST-1 (Boehringer Mannheim, Germany), (slightly red) to Formozan (dark red).

2. Macrophages according to claim 1, which present, on their surface, antigen MHC-II with a mean intensity of about 100 to about 400, such as determined by immunofluorescence staining and flow cytometry analysis.

3. Macrophages according to any one of claims 1 or 2, which are substantially devoid of surface antigen CD83, such as determined by immunofluorescence staining and flow cytometry analysis.

4. Macrophages according to any one of claims 1 to 3, which present adherent properties such as determined by the following test :
the macrophages are cultured for 2 h in culture medium (I.M.D.M. or R.P.M.I.) on plastic flasks and the percentage (%) of adherent cells is quantified for instance by microscopic analysis.

5. Macrophage culture wherein :
- about 20% to about 100% of the macrophages present antigen CD14 on their surface,
- about 20% to about 100% of the macrophages present antigen CD64 on their surface,
- about 80% to about 100% of the macrophages present antigen MHC-II on their surface,
- about 70% to about 100% of the macrophages present adherent properties,
- about 30% to about 100% of the macrophages present a phagocytosis property,
each macrophage having the above-mentioned properties being such that said properties are expressed according to the intensities as specified in any one of claims 1 to 4.

6. Process for preparing a composition comprising macrophages according to any one of claims 1 to 5, comprising the culture of mononuclear cells in a culture medium containing histamine or histamine agonist and a H₂ antagonist, in combination or not with "additional" GM-CSF.

7. Process according to claim 6, wherein the culture medium contains histamine and cimetidine or a H₂ antagonist without "additional" GM-CSF, histamine being present at a concentration of about 10⁻² M to about 10⁻⁶ M, preferably of about 10⁻⁴ M, and cimetidine or the H₂ antagonist being present at a concentration of about 10⁻⁴ M to about 10⁻⁹ M, preferably of about 10⁻⁶ M.

8. Process according to claim 6, wherein the culture medium contains histamine and cimetidine or a H₂ antagonist, in combination with "additional" GM-CSF, histamine being present at a concentration of about 10⁻² M to about 10⁻⁶ M, preferably of about 10⁻⁴ M, cimetidine or the H₂ antagonist being present at a concentration of about 10⁻⁴ M to about 10⁻⁹ M, preferably of about 10⁻⁶ M, and additional GM-CSF being present at a concentration of about 50 U/ml to about 2000 U/ml, preferably of about 500 U/ml.

9. Process according to any one of claims 6 to 8, comprising :
- isolation of leukocytes, from healthy donors or from patients, from peripheral blood by apheresis and removal platelets and anticoagulant from the apheresis product,
- isolation of mononuclear cells (monocytes + lymphocytes) from red cells and granulocytes in order to have less than 10% granulocytes and less than 5% red cells,
- culture of the mononuclear cells obtained at the previous stage by placing them in an appropriate culture medium containing histamine or an agonist of histamine, an H₂ antagonist, such as cimetidine, in combination or not with GM-CSF, for a time sufficient to obtain differentiated macrophages, preferably for about 5 to 15 days, and possibly separating the macrophages from the lymphocytes, and recovering the macrophages or the macrophages and lymphocytes.

10. Process according to any one of claims 6 to 9, wherein the culture medium of macrophages is added
- with crude antigens, for instance autologous tumor membrane, killed tumoral cells, bacterial capsides, viral homogenates cleared from nucleic acids,
- specific peptides against which an immune response is desired,
- cDNA or genetic material linked to vectors (for example gluconated polylysine) to allow transfection of the macrophage with material coding for the relevant peptide or protein to be presented on the macrophage membrane and against which an immune response is desired,
- or bispecific antibodies targeting on the one side, FcγRI (CD64) surface antigen of the macrophage and, on the other side, a relevant antigen against which an immune response is desired.

11. Macrophages liable to be obtained according to the process of any one of claims 6 to 10.

12. Pharmaceutical compositions containing as active substance, macrophages according to any one of claims 1 to 5 or 11.

13. Cellular vaccine compositions containing as active substance, macrophages according to any one of claims 1 to 5 or 11.

14. Medium containing elements necessary for the growth and differentiation of monocytes into macrophages according to claims 1 to 5 or 11, and in addition containing histamine, cimetidine in combination or not with GM-CSF.

15. Cell processor or kit containing :
- means for the recovery of lymphocytes and monocytes free of contaminants,
- appropriate buffer and wash solutions and possibly appropriate means for the conservation of macrophages,
- means for preparing a culture for the monocytes and possibly the lymphocytes and containing histamine, cimetidine or a H₂ antagonist in combination or not with GM-CSF,
- possibly means for transfection of cultured cells and means for targeting antigens to macrophages.

16. Cell processor or kit according to claim 15, containing :
- means for recovering and centrifuging blood to obtain a leukocyte concentrate,
- means for separating lymphocytes and monocytes from the other white cells and for eliminating the contaminating red cells,
- culture medium for macrophages and possibly lymphocytes with complements and particularly histamine and cimetidine or a H₂ antagonist, in combination or not with GM-CSF,
- appropriate means for the conservation of macrophages,
- appropriate buffer and wash solution.

17. Products containing macrophages according to any one of claims 1 to 5 or 11, and lymphocytes, as a combined preparation for simultaneous, separate or sequential use in cell therapy.

18. Products according to claim 17, characterized in that they contain the macrophages and the lymphocytes in a ratio of at least 20% to 50% of macrophages expressed in cell number.

19. Bispecific antibodies liable to recognize an antigen of a macrophage according to any one of claims 1 to 5 or 11 and an antigen of a tumoral cell or of a pathogen which is to be targeted to said macrophage.

20. Method for the clinical treatment, comprising the administration of an appropriate amount of macrophages according to any one of claims 1 to 5 or 11, and preferably in an amount of about 10⁸ to about 5x10⁹ macrophages.

21. Method according to claim 20 for the treatment of any disorder, comprising the administration of lymphocytes in an amount of about 4x10⁹ to about 10x10⁹ lymphocytes.

22. Use of an agonist of histamine, in particular histamine, and a H₂ antagonist, in particular cimetidine, in combination or not with GM-CSF, for the preparation of macrophages having the following properties :
- they present on their surface :
* antigen CD14 with a mean intensity of about 20 to about 200,
* antigen CD64 with a mean intensity of about 20 to about 200,
- they are substantially devoid of the surface antigens CD1a and CD1c,
the presence and mean intensities respectively of CD14, CD64 and the absence of CD1a and CD1c being for instance determined by immunofluorescence staining and flow cytometry analysis,
- they present a phagocytosis property such as determined by the following test:
said phagocytosis capacity being evaluated by an uptake of formalin fixed yeast, for example by culturing macrophages for 2 hours to select adherent cells, adding yeast in 1/10 macrophages/yeast ratio and incubating at 37°C, 5% CO₂ atmosphere for 2-3 hours fixing by the May-Grünwald-Giemsa (MGG) staining, and the percentage of phagocytic macrophages being quantified for instance by microscopic analysis,
- they have the property of stimulating the proliferation of allogenic lymphocytes such as determined by the following test :
allogenic primary mixed lymphocytes reaction (MLR) was carried out in 96-well microtiter plates by adding different numbers (2x10³ to 2x10⁵ in 100 µl medium/well) of macrophages to 2x10⁵ in 100 µl medium/well of allogenic T cells purified from buffy coats and after 5 days incubation at 37°C, cell proliferation was assessed by a colorimetric method, such as the cleavage of tetrazolium salt WST-1 (slightly red) to Formozan (dark red).
